# EUROPEAN PATENT SPECIFICATION

(11) **EP 0 331 374 B1**
(45) Date of publication and mention of the grant of the patent: **16.02.1994**
(21) Application number: 89301866.3
(22) Date of filing: 24.02.1989
(51) Int. Cl.: A61K 31/415

(54) **4-substituted imidazole derivatives useful in perioperative care**
Verwendung von 4-substituierten-Imidazol-Derivaten in der perioperativen Medikation
Utilisation de dérivés imidazoles-4-substitués dans les soins périopératifs

(30) Priority: 29.02.1988 GB 8804683
(43) Date of publication of application: 06.09.1989
(73) Proprietor: ORION-YHTYMÄ OY, 02101 Espoo (FI)
(72) Inventor: Maze, Mervyn, Portola Valley California 92045 (US); Scheinin, Mika, SF-21000 Naantali (FI)
(74) Representative: Sexton, Jane Helen

(56) References cited:
- EP-A- 0 187 471
- EP-A- 0 270 267
- DRUGS OF THE FUTURE, vol. 12, no. 11, 1987, pages 1021-1022; "Medetomidine"
- J. AUTON. PHARMAC., vol. 5, 1986, pages 275-284; J.-M. SAVOLA et al.: "Evidence for medetomidine as a selective and potent agonist at alpha2-adrenoreceptors"
- ACTA VET. SCAND., vol. 29, 19th October 1988, pages 109-116; K. BERGSTRÖM: "Cardiovascular and pulmonary effects of a new sedative/analgesic (medetomidine) as a preanaesthetic drug in the dog"
- ACTA VET. SCAND., vol. 29, 19th October 1988, pages 35-41; B. KOMMONEN: "The DC-recorded dog electroretinogram in ketamine - medetomidine anaesthesia"
- ANESTHESIOLOGY, vol. 69, no. 6, December 1988, pages 818-823; I.S. SEGAL et al.: "Dexmedetomidine diminishes halothane anesthetic requirements in rats through a postsynaptic alpha2 adrenergic receptor"
- J. VET. PHARMACOL. THERAP., vol. 10, 1987, pages 319-323; D. STENBERG et al.: "Sedative action of the alpha2-agonist medetomidine in cats"
- Anaesthesia and Analgesia, 63, p. 640-646 (1984)
- British J. of Anaesthesia, 53, p. 837-839 (1981)

## Description

This invention relates to perioperative use of racemic and dextro-4-[1(2,3-dimethylphenyl)ethyl]-1H-imidazole and their non-toxic pharmaceutically acceptable salts. In the presence of these 4-substituted imidazole compounds, less anesthetic agent is required to achieve a given level of surgical anesthesia. These compounds also decrease circulating catecholamines thereby reducing the stress to which the cardiovascular system is exposed in the perioperative period.

Racemic 4-[1-(2,3-dimethylphenyl)ethyl]-1H-imidazole, which is known under the name medetomidine and which has the formula 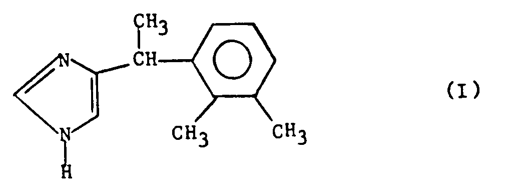
is a selective and potent α₂-adrenoreceptor agonist. It has earlier been disclosed e.g. in the European Patent Publication No. 72615 as an antihypertensive agent and in the European Patent Publication No. 187471 as a veterinary sedative-analgesic agent.

It has further been observed that this compound also possesses anxiolytic effects and can therefore be used in the treatment of general anxiety, panic disorder and various kinds withdrawal symptoms.

As the compound of formula (I) possesses one asymmetric carbon atom this compound, which is a racemic mixture of two optically active stereoisomers, can be split up into its d- and l-enantiomers. In particular the d-enantiomer has been found to exhibit surprisingly high selectivity and potency as an α₂-adrenoreceptor agonist.

Clonidine, a known antihypertensive drug (US patent 3202660), has recently been reported to exhibit valuable properties for the use in the perioperative field. These observations have been reported in two papers:
Flacke J W, Bloor B C, Flacke W E, Wong D, Dazza S, Stead S W, Laks H: Reduced narcotic requirement by clonidine with improved hemodynamic and adrenergic stability in patients undergoing coronary bypass surgery; Anesthesiology 67: 909-917, 1987 and Ghignone M, Calvillo O, Quintin L: Anesthesia and hypertension: The effect of clonidine on perioperative hemodynamics and isoflurane requirements; Anesthesiology 67:901-908, 1987.

The first paper relates to a study of patients undergoing coronary artery bypass graft operations. Clonidine was given as premedication as well as intraoperatively. The administration of clonidine caused reduction in the anesthesia requirement and decreased plasma concentration of catecholamines. The second paper relates to a study of the influence of clonidine premedication on perioperative hemodynamics in hypertensive patients. Clonidine decreased the lability of heart rate and blood pressure in hypertensive patients and the requirement for anesthesia decreased by about 40%.

It has now been found that medetomidine and the d-enantiomer of 4-[1-(2,3-dimethylphenyl)ethyl]-1H-imidazole and their pharmceutically acceptable salts exhibit properties which make them useful in the perioperative care of mammals. The d-enantiomer is especially useful in this respect.

Thus the present invention provides use of a compound which is racemic or dextro 4-[1-(2,3-dimethylphenyl)ethyl]-1H-imidazole or a non-toxic pharmaceutically acceptable salt thereof, in the manufacture of a medicament for perioperative treatment of mammals to reduce the responses of the autonomic nervous system to stressful stimuli during the operation.

The present invention also provides a pharmaceutical composition comprising dextro 4-[1-(2,3-dimethylphenyl)ethyl]-1H-imidazole or a non-toxic pharmaceutically acceptable salt thereof and a pharmaceutically acceptable carrier or diluent.

Medetomidine and its d-enantiomer essentially reduce the anesthetic requirement and other stress factors during the operation. The MAC-sparing (MAC = minimum alveolar concentration) and cardiovascular effects of these compounds in halothane-anesthetized dogs are reported as follows:
Anesthesia was induced by mask inhalation of halothane in oxygen in male beagles (8-11 kg). Following tracheal intubation, ventilation was controlled to maintain normocarbia (CO₂ _{ET} -4.5 %). Catheters were inserted percutaneously for: 1) intraarterial blood gas determination and pressure recording (femoral artery); 2) pulmonary arterial and central venous pressure monitoring and cardiac output (thermodilution) assessment; and 3) intravenous fluid and drug administration. End-tidal halothane and CO₂ concentrations (infrared analysis), heart rate and rhythm (lead II of the EKG), systemic arterial pressure, central venous pressure, and pulmonary arterial pressure were continuously displayed and recorded. Core temperature was maintained at 37°C with insulating blankets and heating lamps. After a 2 h equilibration period, the MAC for halothane was determined as previously described (Nicholls E A, Louis G L, Prokocimer P G, Maze M: Halothane anesthetic requirements are not affected by aminophylline treatment in rats and dogs. Anesthesiology 65:637-641, 1986) and baseline hemodynamic function (mean arterial blood pressure, heart rate, central venous pressure, pulmonary arterial diastolic pressure, pulmonary artery occluded pressure, cardiac output and derived systemic vascular resistance) was assessed. Medetomidine in the DL- (n-7), D- (n-5), and L-forms were administered in separate experiments at each of three doses (1, 3 and 10 µg/kg) via the right arterial port over 15 min while maintaining the dog at its individual MAC for halothane. Ten minutes after the termination of the infusion, (at which time the profile was stable) hemodynamic function was reassessed. Also at this time arterial blood was sampled for 1) measurement of gas tensions and acid/base status; and 2) circulating norepinephrine. MAC determination was then repeated following which the successive dose of medetomidine was given and the routine repeated. Data were compared by ANOVA for repeated measurements and subsequently by paired t-test with Bonferroni correction. A p value of < 0.05 was considered the level for statistical significance. MAC for halothane significantly decreased following DL-medetomidine administration in a dose-dependent fashion to the extent that at the highest dose (10 µg/kg), the halothane MAC was less than <0.1 %.

This effect could be mimicked by the D-isomer while the L-isomer was without effect. Neither isomer changed the mean arterial pressure while only the D-isomer significantly decreased heart rate and cardiac output.

Results: MAC for halothane significantly decreased following medetomidine administration in a dose-dependent fashion. The D-enantiomer displayed the same MAC-sparing effect (Table 1).

**Table 1**

| Effect of medetomidine and the d-enantiomer on MAC | | |
|---|---|---|
| Dose µg/kg | end-tidal halothane (% v/v) | |
| | medetomidine X ± SD (Standard deviation) | d-enantiomer X ± SD |
| 0 | 0.90 ± .07 | 1.00 ± .16 |
| 1 | 0.73 ± .21 | 0.65 ± .14 |
| 3 | 0.35 ± .19 | 0.40 ± .09 |
| 10 | 0.05 ± .09 | 0.11 ± .07 |

Table 2 and 3 disclose the decreased heart rate and cardiac output resulting from the administration of the compounds.

**Table 2**

| Effect of medetomidine and the d-enantiomer on heart rate (beats per minute) at 1 MAC halothane | | |
|---|---|---|
| Dose µg/kg | medetomidine X ± SD (bpm) | d-enantiomer X ± SD (bpm) |
| 0 | 111 ± 15 | 116 ± 6 |
| 1 | 89 ± 14 | 86 ± 9 |
| 3 | 76 ± 10 | 64 ± 9 |
| 10 | 63 ± 10 | 57 ± 6 |

**Table 3**

| Effect of medetomidine and the d-enantiomer on cardiac output at 1 MAC halothane | | |
|---|---|---|
| Dose µg/kg | medetomidine X ± SD (1/min) | d-enantiomer X ± SD (1/min) |
| 0 | 2.25 ± .85 | 2.61 ± .36 |
| 1 | 1.43 ± .53 | 1.71 ± .73 |
| 3 | 0.93 ± .22 | 1.07 ± .14 |
| 10 | 0.86 ± .25 | 0.97 ± .09 |

The compounds can be administered i.v., i.m. or orally The preferably dose range is 1 µg/kg - 10 µg/kg.

Medetomidine as premedication before dental surgery:
Racemic medetomidine (50 µg, intravenously) was given to ten healthy subjects as premedication 20-30 min before surgical third molar extraction in a random-order, cross-over, placebo-controlled phase II clinical investigation. Local anaesthesia was with lidocaine/ epinephrine. Each patient had two operations separated by at least three weeks. Medetomidine was rated as clearly superior to placebo by both the patients and the dental surgeon (see Table 4). No adverse effects were noted, neither in hemodynamic parameters, clinical chemistry tests nor in the patients' subjective reports.

**Table 4**

| Effectiveness of medetomidine as premedication before surgical third molar extraction (10 patients, bilateral operations) | | | |
|---|---|---|---|
| | Racemic medetomidine better | both drugs equal | placebo better |
| Patients' preference | 8 | 2 | 0 |
| Effectiveness assessed by dental surgeon | 10 | 0 | 0 |

## Claims

1. Use of a compound which is racemic or dextro 4-[1-(2,3-dimethylphenyl)ethyl]-1H-imidazole or a non-toxic pharmaceutically acceptable salt thereof, in the manufacture of a medicament for perioperative treatment of mammals to reduce the responses of the autonomic nervous system to stressful stimuli during the operation.

2. Use of a compound according to claim 1 which is dextro 4-[1-(2,3-dimethylphenyl)ethyl]-1H-imidazole or a non-toxic pharmaceutically acceptable salt thereof.

3. Use of a compound according to claim 1 or 2 in which the medicament is for use as anxiolytic analgesic premedication prior to an operation with or without local or general anaesthesia.

4. Use of a compound according to any one of claims 1 to 3 in which the medicament is for use preoperatively as sedative analgesic to potentiate the effect of anaesthetics and thus to reduce the anaesthetic requirement.

## Patentansprüche

1. Verwendung einer Verbindung, die ein racemisches oder rechtsdrehendes 4-[1-(2,3-Dimethylphenyl)ethyl]-1H-imidazol oder ein nicht-toxisches pharmazeutisch akzeptierbares Salz davon ist, beim Herstellen eines Medikamentes zur perioperativen Behandlung von Säugern zur Verringerung der Reaktion des autonomen Nervensystemes auf Streßreize während der Operation.

2. Verwendung einer Verbindung nach Anspruch 1, die rechtsdrehendes 4-[1-(2,3-Dimethylphenyl)ethyl]-1H-imidazol oder ein nichttoxisches pharmazeutisch akzeptierbares Salz davon ist.

3. Verwendung einer Verbindung nach Anspruch 1 oder 2, bei der das Medikament zur Benutzung als anxiolytische analgetische Prämedikation vor einer Operation mit oder ohne lokaler Betäubung oder allgemeiner Anästhesie dient.

4. Verwendung einer Verbindung nach einem der Ansprüche 1 bis 3, bei der das Medikament zur präoperativen Benutzung als beruhigendes Analgetikum zum Verstärken des Effektes von Anästhesiemitteln und somit zum Verringern der anästhetischen Anforderung dient.

## Revendications

1. Utilisation d'un composé racémique ou dextrogire du 4-|1-(2,3-dimétnylphényl)éthyl|-1H-imidazole ou un sel non-toxique pharmaceutiquement acceptable de ce composé, pour la préparation d'un médicament destiné au traitement périopératif de mammifères afin de réduire les réponses du système nerveux automatique aux stimuli stressants pendant l'opération.

2. Utilisation d'un composé selon la revendication 1, caractérisée en ce que le composé est un composé dextrogire du 4-|1-(2,3-diméthylphényl)éthyl|-1H-imidazole ou un sel non-toxique pharmaceutiquement acceptable de celui-ci.

3. Utilisation d'un composé selon la revendication 1 ou 2, caractérisée en ce que le médicament est utilisé comme prémédication anxiolytique analgésique avant une opération avec ou sans anesthésie locale ou générale.

4. Utilisation d'un composé selon l'une quelconque des revendications 1 à 3, caractérisée en ce que le médicament est utilisé avant l'opération comme analgésique sédatif afin de potentialiser l'effet de l'anesthésique et ainsi réduire le besoin en anesthésique.
